(19) Europäisches Patentamt
European Patent Office
Office européen des brevets

(11) Veröffentlichungsnummer: **0 375 977 B1**

# (12) EUROPÄISCHE PATENTSCHRIFT

(45) Veröffentlichungstag der Patentschrift: **02.02.94**

(51) Int. Cl.$^5$: **C07D 231/38**, A61K 7/13

(21) Anmeldenummer: **89122091.5**

(22) Anmeldetag: **30.11.89**

Die Akte enthält technische Angaben, die nach dem Eingang der Anmeldung eingereicht wurden und die nicht in dieser Patentschrift enthalten sind.

## (54) Oxidationshaarfärbemittel mit einem Gehalt an Diaminopyrazolderivaten und neue Diaminopyrazolderivate.

(30) Priorität: **24.12.88 DE 3843892**

(43) Veröffentlichungstag der Anmeldung:
**04.07.90 Patentblatt 90/27**

(45) Bekanntmachung des Hinweises auf die Patenterteilung:
**02.02.94 Patentblatt 94/05**

(84) Benannte Vertragsstaaten:
**DE ES FR GB IT**

(56) Entgegenhaltungen:
**DE-A- 2 160 317**
**DE-A- 2 160 318**

**JUSTUS LIEBIGS ANNALEN DER CHEMIE, Band 707, 1967, Seiten 141-146, Weinheim, DE; H. DORN et al.: "Über die elektrophile Substitution von 3(5)-Amino-pyrazol"**

**JUSTUS LIEBIGS ANNALEN DER CHEMIE, Band 717, 1968, Seiten 118-123, Weinheim, DE; H. DORN et al.: "Synthese und Methylierung von 1H-Pyrazolo[3.4-b]pyrazinen, einer neuen Klasse von Purin-Antagonisten"**

**CHEMISCHE BERICHTE, Band 101, Nr. 9, 1968, Seiten 3265-3277, Weinheim, DE; H. DORN et al.: "Potentielle Cytostatica. XVI. Bicyclische Systeme aus Acetessigester und 5-Amino-1-methyl-,5-Amino-1-benzyl- sowie 3(5)-Amino-pyrazol"**

(73) Patentinhaber: **Wella Aktiengesellschaft**
**Berliner Allee 65**
**D-64295 Darmstadt(DE)**

(72) Erfinder: **Clausen, Thomas, Dr.**
**Ernst-Pasqué-Strasse 35 A**
**D-6146 Alsbach(DE)**
Erfinder: **Kern, Ute, Dr.**
**Gehmerweg 9**
**D-6100 Darmstadt-Arheilgen(DE)**
Erfinder: **Neunhoeffer, Hans, Prof. Dr.**
**Auf dem Sand 1**
**D-6109 Mühltal(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Gegenstand der Erfindung sind Mittel zur oxidativen Färbung von Haaren auf der Basis von 3,4- oder 4,5-Diaminopyrazolderivaten als Entwicklersubstanz sowie neue 3,4- oder 4,5-Diaminopyrazolderivate.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Als Entwicklersubstanzen werden insbesondere 2,5-Diaminotoluol, 2,5-Diaminophenylethylalkohol, p-Aminophenol und 1,4-Diaminobenzol eingesetzt. Von den vorzugsweise verwendeten Kupplersubstanzen sind Resorcin, 4-Chlorresorcin, 1-Naphthol, 3-Aminophenol, 5-Amino-2-methylphenol und Derivate des m-Phenylendiamins zu nennen.

An Oxidationsfarbstoffe, die zur Färbung menschlicher Haare Verwendet werden, sind zahlreiche besondere Anforderungen gestellt. So müssen sie in toxikologischer und dermatologischer Hinsicht unbedenklich sein und Färbungen in der gewünschten Intensität ermöglichen. Ferner wird für die erzielten Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben. Außerdem ist es erforderlich, daß durch Kombinationen geeigneter Entwickler- und Kupplerkomponenten eine breite Palette verschiedener Farbnuancen erzeugt werden kann. Zur Erzielung natürlicher und besonders modischer Nuancen im Rotbereich wird vor allem 4-Aminophenol, allein oder im Gemisch mit anderen Entwicklersubstanzen, in Kombination mit geeigneten Kupplersubstanzen eingesetzt.

Gegen den für den Rotbereich der Farbskala bisher hauptsächlich eingesetzten Entwickler 4-Aminophenol wurden in letzter Zeit Bedenken in bezug auf die physiologische Verträglichkeit erhoben, während die in neuerer Zeit empfohlenen Entwicklersubstanzen, wie zum Beispiel Pyrimidinderivate, in färberischer Hinsicht nicht völlig zufriedenstellen können. Die in der DE-OS 2 160 317 beschriebenen Pyrazolderivate, wie zum Beispiel das 3-Amino-1-phenyl-2-pyrazolon-5, färben Haare nur in sehr geringen, für die Haarfärbepraxis unbrauchbaren, Farbtiefen an.

Es bestand daher die Aufgabe, ein Oxidationshaarfärbemittel auf der Basis einer Entwicklersubstanz/Kupplersubstanz-Kombination zur Verfügung zu stellen, in dem eine Entwicklersubstanz für den Rotbereich enthalten ist, welche physiologisch sehr gut verträglich ist und mit üblichen Kupplersubstanzen das Haar in brillianten roten Farbtönen mit einer hohen Farbtiefe färbt.

Hierzu wurde nun gefunden, daß durch ein Mittel zur oxidativen Färbung von Haaren auf der Basis einer Entwicklersubstanz-Kupplersubstanz-Kombination, welche als Entwicklersubstanz ein Diaminopyrazol der allgemeinen Formel (I)

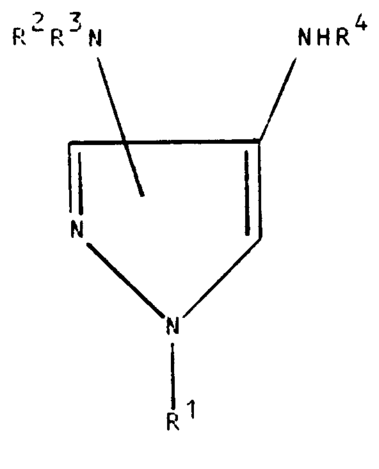

(I),

in der $R^1$, $R^2$ und $R^4$ gleich oder verschieden sind und Wasserstoff, Alkyl mit 1 bis 4 Kohlenstoffatomen, Hydroxyalkyl mit 2 bis 4 Kohlenstoffatomen. Benzyl oder Phenyl bedeuten, sowie $R^3$ Wasserstoff, Alkyl mit 1 bis 4 Kohlenstoffatomen oder Hydroxyalkyl mit 2 bis 4 Kohlenstoffatomen darstellt, oder dessen physiologisch verträgliche, wasserlösliche Salze enthält, die gestellte Aufgabe in hervorragender Weise gelöst wird.

In dem Haarfärbemittel sollen die Entwicklersubstanzen der Formel (I), von denen das 3(5),4-Diaminopyrazol, das 4,5-Diamino-1-methylpyrazol sowie das neue 4,5-Diamino-1-benzylpyrazol bevorzugt sind, in

einer Menge von etwa 0,01 bis 3,0 Gewichtsprozent, vorzugsweise in einer Menge von 0,1 bis 2,5 Gewichtsprozent, enthalten sein.

Obwohl die vorteilhaften Eigenschaften der hier beschriebenen neuen Entwicklersubstanzen es nahelegen, diese als alleinige Entwicklersubstanz zu verwenden, ist es selbstverständlich auch möglich, die Entwicklersubstanzen der Formel (I) gemeinsam mit bekannten Entwicklersubstanzen, wie zum Beispiel 1,4-Diaminobenzol, 2,5-Diaminotoluol oder 2,5-Diaminophenylethylalkohol, einzusetzen.

Als Kupplersubstanzen kommen als Bestandteil des hier beschriebenen Haarfärbemittels vorzugsweise Resorcin, 4-Chlorresorcin, 4,6-Dichlorresorcin, 2-Methylresorcin, 2-Amino-4-(2'-hydroxyethyl)amino-anisol, 2,4-Diaminobenzylalkohol, 2,4-Diaminophenylethylalkohol, m-Phenylendiamin, 5-Amino-2-methylphenol, 2,4-Diaminophenoxyethanol, 4-Amino-2-hydroxyphenoxyethanol, 1-Naphthol, 3-Aminophenol, 3-Amino-2-methylphenol, 4-Hydroxy-1,2-methylendioxybenzol, 4-Amino-1,2-methylendioxybenzol, 4-(2'-Hydroxyethyl)-amino-1,2-methylendioxybenzol, 2,4-Diamino-phenetol,2,4-Diamino-5-methylphenetol, 4-Hydroxyindol, 3-Amino-5-hydroxy-2,6-dimethoxypyridin und 3,5-Diamino-2,6-dimethoxypyridin in Betracht.

Die Kuppler- und Entwicklersubstanzen können in dem Haarfärbemittel jeweils einzeln oder im Gemisch miteinander enthalten sein.

Die Gesamtmenge der in dem hier beschriebenen Haarfärbemittel enthaltenen Entwicklersubstanz-Kupplersubstanz-Kombination beträgt 0,1 bis 5,0 Gewichtsprozent, wobei eine Menge von 0,5 bis 4,0 Gewichtsprozent, bevorzugt ist.

Die Entwicklerkomponenten werden im allgemeinen in etwa äquimolaren Mengen, bezogen auf die Kupplerkomponenten, eingesetzt. Es ist jedoch nicht nachteilig, wenn die Entwicklerkomponente diesbezüglich in einem gewissen Überschuß oder Unterschuß vorhanden ist.

Weiterhin kann das erfindungsgemäße Haarfärbemittel zusätzlich andere Farbkomponenten, beispielsweise 6-Amino-2-methyl-phenol und 2-Amino-5-methyl-phenol, sowie ferner übliche direktziehende Farbstoffe, zum Beispiel Triphenylmethanfarbstoffe wie Diamond Fuchsine (C.I. 42 510) und Leather Ruby HF (C.I. 42 520), aromatische Nitrofarbstoffe wie 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethyl)amino-nitrobenzol und 2-Methylamino-5-bis-(2'-hydroxyethyl)amino-nitrobenzol, Azofarbstoffe wie Acid Brown 4 (C. I. 14 805) und Dispersionsfarbstoffe wie beispielsweise 1,4-Diaminoanthrachinon und 1,4,5,8-Tetraaminoanthrachinon enthalten. Die Haarfärbemittel können diese Farbkomponenten in einer Menge von etwa 0,1 bis 4,0 Gewichtsprozent enthalten.

Selbstverständlich können die Kuppler- und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in dem Haarfärbemittel noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.

Die Zubereitungsform des neuen Haarfärbemittels kann beispielsweise eine Lösung, insbesondere eine wäßrige oder wäßrig-alkoholische Lösung, sein. Die besonders bevorzugten Zubereitungsformen sind jedoch eine Creme, ein Gel oder eine Emulsion.

Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol, Isopropanol, Glycerin, oder Glykole wie 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkylbenzolsulfonate, Alkyltrimethylammonoiumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Haarfärbemittel schwach sauer, neutral oder alkalisch reagieren. Insbesondere weist es einen pH-Wert von 8,0 bis 11,5 auf, wobei die Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden.

Für die Anwendung zur oxidativen Färbung von Haaren vermischt man das vorstehend beschriebene Haarfärbemittel unmittelbar vor dem Gebrauch mit einem Oxidationsmittel und trägt eine für die Haarfärbebehandlung ausreichende Menge, je nach Haarfülle, im allgemeinen etwa 60 bis 200 g, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin oder Natriumborat in Form einer 3 bis 12 prozentigen, vorzugsweise 6 prozentigen, wäßrigen Lösungen in Betracht. Wird eine 6 prozentige Wasserstoffperoxid-Lösung als Oxidationsmittel verwendet, so beträgt das Gewichtsverhältnis zwischen Haarfärbemittel und Oxidationsmittel 5 : 1 bis 1 : 2, vorzugsweise jedoch 1 : 1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die Herstellung der erfindungsgemäß verwendeten Entwicklersubstanzen ist zum Teil bekannt. So wird zum Beispiel das 3(5),4-Diaminopyrazol[1)] und das 4,5-Diamino-1-methylpyrazol[2)] in der Literatur beschrieben: 1) H. Dorn et al., Liebigs Ann. Chem. 707 (1967) 141 - 146; 2) H. Dorn et al., Liebigs Ann. Chem. 717 - (1968) 118 - 123.

3,4- und 4,5-Diamino-1-methylpyrazol lassen sich aus dem in Literatur 1) beschriebenen 3(5)-Amino-4-nitropyrazol durch Alkylierung und anschließende Reduktion darstellen.

Die neuen Verbindungen der nachfolgenden Formeln (II) und (III) sowie das neue 3,4-Diamino-1-methylpyrazol können auf verschiedenen Wegen synthetisiert werden: 3,4-Diamino-1-benzylpyrazol läßt sich analog der oben beschriebenen Methylverbindungen durch Benzylierung und nachfolgende Reduktion darstellen. Das 4,5-Diamino-1-benzylpyrazol wird aus dem 5-Amino-1-benzylpyrazol (H. Dorn et al., Chem. Ber. 101 (1968) 3265 - 3277) durch Nitrosierung und anschließende Reduktion hergestellt.

Die an den Aminogruppen alkylierten Derivate lassen sich alle, wie in den Beispielen beschrieben, durch Alkylierung der intermediär gebildeten Aminonitropyrazole und nachfolgende Reduktion der Nitrogruppen darstellen.

Die Salze der Verbindungen der Formel (I) sind durch Umsetzung mit organischen oder anorganischen Säuren oder Basen erhältlich.

Die Entwicklersubstanzen der Formel (I) sollen in dem Haarfärbemittel entweder als freie Basen oder in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden. Die Verbindungen der Formel (I) sind gut in Wasser löslich, sie weisen außerdem eine ausgezeichnete Lagerstabilität, insbesondere als Bestandteil der hier beschriebenen Haarfärbemittel auf.

Das erfindungsgemäße Haarfärbemittel mit einem Gehalt an 3,4- oder 4,5-Diaminopyrazolderivaten als Entwicklersubstanzen ermöglicht Haarfärbungen mit ausgezeichneter Farbechtheit, insbesondere was die Licht-, Wasch- und Reibechtheit anbetrifft, und die Haarfärbungen lassen sich mit Reduktionsmitteln wieder abziehen.

Von besonderer Bedeutung ist weiterhin der durch die Verwendung der 3,4- oder 4,5-Diaminopyrazole in dem Haarfärbemittel gemäß vorliegender Anmeldung in toxikologischer und dermatologischer Hinsicht erzielte Fortschritt. So sind die Verbindungen 3(5),4-Diaminopyrazol und 4,5-Diamino-1-methylpyrazol nicht mutagen.

Hinsichtlich der färberischen Eigenschaften bieten die erfindungsgemäßen Haarfärbemittel Möglichkeiten, die weit über einen Ersatz der üblicherweise verwendeten 4-Aminophenole hinausgehen. So lassen sich brillante Rottöne mit außerordentlicher Farbtiefe erzeugen, wie sie mit den gängigen Farbkomponenten nicht zu erzielen sind. Neben dieser Anwendung im hochmodischen Bereich können aber auch durch die Verwendung in Kombination mit geeigneten Kupplungskomponenten natürliche Farbtöne erzeugt werden, ohne daß eine weitere Entwicklungskomponente vom Typ der p-Phenylendiamine erforderlich wäre.

Die sehr guten färberischen Eigenschaften der Haarfärbemittel gemäß der vorliegenden Anmeldung zeigen sich weiterhin darin, daß diese Mittel eine Anfärbung von ergrauten, chemisch nicht vorgeschädigten Haaren problemlos und mit guter Deckkraft ermöglichen.

Gegenstand der vorliegenden Patentanmeldung sind ferner neue Diaminopyrazolderivate und zwar (A) 3,4-Diamino-1-methylpyrazol, (B) Diaminopyrazolderivate der allgemeinen Formel (II)

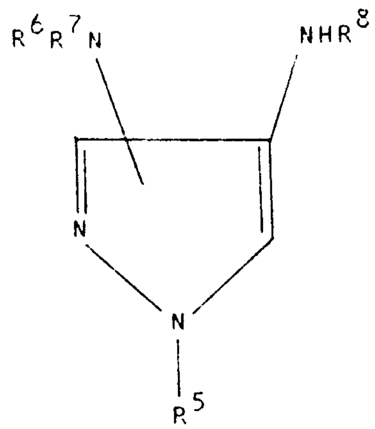

(II),

in der $R^5$ ein Benzylrest ist, $R^6$ und $R^8$ gleich oder verschieden sind und Wasserstoff, Alkyl mit 1 bis 4 Kohlenstoffatomen, Hydroxyalkyl mit 2 bis 4 Kohlenstoffatomen, Benzyl oder Phenyl bedeuten und $R^7$ Wasserstoff, Alkyl mit 1 bis 4 Kohlenstoffatomen oder Hydroxyalkyl mit 2 bis 4 Kohlenstoffatomen darstellt, wobei insbesondere 4,5-Diamino-1-benzylpyrazol, 3,4-Diamino-1-benzylpyrazol, 4-Amino-1-benzyl-3-(2'-hydroxyethyl)amino-pyrazol und 4-Amino-1-benzyl-3-benzylaminopyrazol zu nennen sind, sowie (C) Diaminopyrazolderivate der allgemeinen Formel (III)

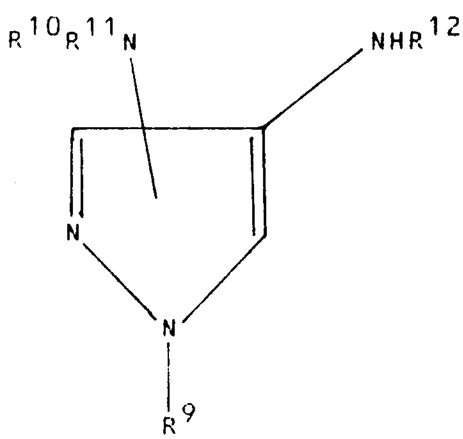

(III),

in der $R^9$ ein Methylrest ist, $R^{10}$ und $R^{12}$ gleich oder verschieden sind und Wasserstoff, Alkyl mit 1 bis 4 Kohlenstoffatomen, Hydroxyalkyl mit 2 bis 4 Kohlenstoffatomen, Benzyl oder Phenyl bedeuten und $R^{11}$ Wasserstoff, Alkyl mit 1 bis 4 Kohlenstoffatomen, Hydroxyalkyl mit 2 bis 4 Kohlenstoffatomen darstellt, unter der Voraussetzung, daß mindestens einer der Reste $R^{10}$ bis $R^{12}$ von Wasserstoff verschieden ist, wobei insbesondere 4-Amino1-methyl-3-methylaminopyrazol und 4-Amino-1-methyl-5-N,N-dimethylaminopyrazol genannt werden.

In den nachstehenden Beispielen soll der Gegenstand der Erfindung näher erläutert werden, ohne ihn auf die Beispiele zu beschränken.

## Herstellungsbeispiele

**Beispiel 1:** Synthese von 3,4-Diamino-1-benzylpyrazol

Stufe 1: Umsetzung von 3(5)-Amino-4-nitropyrazol mit Benzylbromid

1,00 g (7,80 mmol) 3(5)-Amino-4-nitropyrazol werden in 30 ml absolutem Dioxan mit 187 mg (7,80 mmol) Natriumhydrid versetzt. Nach beendeter Wasserstoffentwicklung werden zu der Reaktionsmischung 1,33 g (7,80 mmol) Benzylbromid zugetropft und das Gemisch wird 17 Stunden lang zum Sieden erhitzt. Sodann wird das Lösungsmittel am Rotationsverdampfer im Vakuum abdestilliert und der Rückstand durch

Säulenchromatographie an Kieselgel mit Toluol/Ether (8 : 1) aufgetrennt.

1. Fraktion:

Man erhält ein braunes Öl, das im Kugelrohr bei 220 Grad Celsius/0,05 Torr destilliert wurde und nach Behandlung mit einem Gemisch aus Chloroform und Tetrachlorkohlenstoff kristallisiert.

Ausbeute:

430 mg (17,9 Prozent der Theorie) 1-Benzyl-3-benzylamino-4-nitropyrazol als leuchtend gelbe Kristalle mit einem Schmelzpunkt von 76 Grad Celsius (Chloroform/Tetrachlorkohlenstoff).
60-MHz-$^1$H-NMR ($CDCl_3$):
δ = 7,71 (s; 1 H)
7,28 (s; 10 H)
6,12 (s; 1 H; mit $D_2O$ austauschbar)
5,03 (s; 2 H)
4,55 - 4,48 ppm (d; 2 H; J = 6 Hz; $-NH-CH_2-$)
Für dieses und alle folgenden NMR-Spektren gilt: s = Singulett, d = Dublett, t = Triplett, m = Multiplett
MS (70 eV): m/e (Prozent) = (308; $M^+$), 290 (15), 105 (24) 91 (100), 55 (24).
Für dies und alle folgenden Massenspektren gilt: Die relative Intensität der Molekülfragmente wird in Prozent angegeben, wobei das Molekülfragment mit der größten Intensität gleich 100 Prozent gesetzt wird.
UV ($CH_2Cl_2$):$\lambda_{max}$ (log ε) = 281 (3,90), 373 nm (4,07).

| $C_{17}H_{16}N_4O_2$ (308,34) | | | |
|---|---|---|---|
| Berechnet: | C 66,22 | H 5,23 | N 18,17 |
| Gefunden: | C 66,40 | H 5,31 | N 18,18 |

2. Fraktion:

1,33 g (78,1 Prozent der Theorie) 3-Amino-1-benzyl-4-nitropyrazol als gelbe Nadeln mit einem Schmelzpunkt von 140 Grad Celsius (Ether).
60-MHz-$^1$H-NMR ( $D_6$ -DMSO):
δ = 8,59 (s; 1 H)
7,28 (s; 5 H)
6,18 (s; 2 H; mit $D_2O$ austauschbar)
5,09 ppm (s; 2 H)
MS (70 eV): m/e (Prozent) = 218 (82, $M^+$), 201 (6), 91 (100), 65 (35).
UV ($CH_2Cl_2$):$\lambda_{max}$ (log ε) = 278 (3,89), 343 nm (3,72).

| $C_{10}H_{10}N_4O_2$ (218,20) | | | |
|---|---|---|---|
| Berechnet: | C 55,05 | H 4,62 | N 25,68 |
| Gefunden: | C 54,80 | H 4,47 | N 25,73 |

Stufe 2: Reduktion von 3-Amino-1-benzyl-4-nitropyrazol

1,00 g (4,58 mmol) 1-Benzyl-3-amino-4-nitropyrazol werden in 100 ml absolutem Methanol mit katalytischen Mengen Palladium/Kohlenstoff (10 prozentig) bei Raumtemperatur und 50 bar hydriert. Nach 17 Stunden ist die Hydrierung beendet und durch die filtrierte Lösung wird 5 Minuten lang Chlorwasserstoffgas geleitet. Die Lösung wird am Rotationsverdampfer im Vakuum auf 1/3 des ursprünglichen Volumens eingeengt und dann solange mit Essigester versetzt, bis ein Niederschlag ausfällt, der anschließend aus Essigester/Methanol umkristallisiert wird.

Ausbeute:

980 mg (76,6 Prozent der Theorie) 3,4-Diamino-1-benzylpyrazol-dihydrochlorid-hydrat als blaßrosa Kristalle mit einem Schmelzpunkt von 139 Grad Celsius (Essigester/Methanol).
60-MHz-$^1$H-NMR ( $D_6$ -DMSO):
$\delta$ = 7,51 (s; 1 H)
7,25 (s; 5 H)
6,82 (s; 8 H; mit $D_2O$ austauschbar)
5,24 ppm (s; 2 H)

| $C_{10}H_{14}Cl_2N_4 * H_2O$ | | | |
|---|---|---|---|
| Berechnet: | C 43,02 | H 5,77 | N 20,06 |
| Gefunden: | C 43,26 | H 5,64 | N 20,23 |

**Beispiel 2:** Synthese von 4-Amino-1-benzyl-3-benzylaminopyrazol-dihydrochlorid

500 mg (1,62 mmol) 1-Benzyl-3-benzylamino-4-nitropyrazol (Beispiel 1, Stufe 1, Fraktion 1) werden in 20 ml 5 normaler methanolischer Salzsäure mit katalytischen Mengen Palladium/Kohlenstoff bei Raumtemperatur und 50 bar hydriert. Nach 17 Stunden ist die Hydrierung beendet und es wird 5 Minuten lang Chlorwasserstoffgas durch die Lösung geleitet. Die Lösung wird am Rotationsverdampfer im Vakuum auf das halbe Volumen eingeengt und dann solange mit Essigester versetzt bis ein farbloser Niederschlag ausfällt, der aus Essigester/Methanol umkristallisiert wird.

Ausbeute:

350 mg (61,5 Prozent der Theorie) 4-Amino-1-benzyl-3-benzylaminopyrazol-dihydrochlorid als farblose Kristalle mit einem Schmelzpunkt von 149 Grad Celsius (Essigester/Methanol).
60-MHz-$^1$H-NMR ( $D_6$ -DMSO):
$\delta$ = 9,10 (s; 5 H; mit $D_2O$ austauschbar)
7,89 (s; 1 H)
7,18 (s; 10 H)
5,10 (s; 2 H)
4,35 ppm (s; 2 H)

| $C_{17}H_{20}Cl_2N_4$ (351,28) | | | |
|---|---|---|---|
| Berechnet: | C 58,13 | H 5,74 | N 15,95 |
| Gefunden: | C 57,82 | H 5,74 | N 16,19 |

**Beispiel 3:** Synthese von 4-Amino-3-(2'-hydroxyethyl)amino-1-benzylpyrazol

Stufe 1: Synthese von N-3-(1-Benzyl-4-nitropyrazolyl) carbaminsäure-$\beta$-bromethylester

2,00 g (9,17 mmol) 3-Amino-1-benzyl-4-nitropyrazol werden in 30 ml absolutem Tetrahydrofuran mit 1,54 g (15,4 mmol) Calciumcarbonat versetzt und auf 60 Grad Celsius erhitzt. In die Lösung werden 2,40 g (12,8 mmol) Chlorameisensäure-$\beta$-bromethylester getropft und das Reaktionsgemisch 6 Stunden lang zum Sieden erhitzt. Das Reaktionsgemisch wird filtriert und sodann das Filtrat am Rotationsverdampfer auf das halbe Volumen eingedampft. Man erhält blaßgelbe Kristalle, die aus Ether umkristallisiert werden.

Ausbeute:

2,31 g (68,2 Prozent der Theorie) N-3-(1-Benzyl-4-nitropyrazolyl) carbaminsäure-$\beta$-bromethylester als blaßgelbe Kristalle mit einem Schmelzpunkt von 102 Grad Celsius (Ether).
60-MHz-$^1$H-NMR ( $D_6$ -DMSO):

| | |
|---|---|
| $\delta$ = | 9,80 (s; 1 H; mit $D_2O$ austauschbar) |
| | 8,90 (s; 1 H) |
| | 7,32 (s; 5 H) |
| | 5,30 (s; 2 H) |
| | 4,50 - 4,21 (t; 2 H) |
| | 3,77 - 3,50 ppm (t; 2 H) |
| MS (70 eV) : m/e (Prozent) = | 370 (6, $M^+$, $^{81}$Br), 368 (6; $M^+$; $^{79}$Br), 324 (3; $^{81}$Br), 322 (3; $^{79}$Br), 231 (3), 91 (100), 65 (23). |
| UV ($CH_2Cl_2$):$\lambda_{max}$ (log $\epsilon$) = | 279 (3,86), 314 nm sh (3,81). |

| $C_{13}H_{13}BrN_4O_4$ (369,20) | | | |
|---|---|---|---|
| Berechnet: | C 42,29 | H 3,55 | N 15,18 |
| Gefunden: | C 42,23 | H 3,28 | N 15,22 |

Stufe 2: Synthese von N-(1-Benzyl-4-nitropyrazolyl)oxazolidin-2-on

400 mg (1,10 mmol) N- 3-(1-Benzyl-4-nitropyrazolyl) carbaminsäure-$\beta$-bromethylester werden in 10 ml 4 normaler Natronlauge 17 Stunden lang bei Raumtemperatur gerührt. Der erhaltene Niederschlag wird aus Essigester umkristallisiert.

Ausbeute:

260 mg (82,0 Prozent der Theorie) N-(1-Benzyl-4-nitropyrazolyl)oxazolidin-2-on als hellgelbe Kristalle mit einem Schmelzpunkt von 120 Grad Celsius (Essigester).
60-MHz-$^1$H-NMR ($CDCl_3$):

| | |
|---|---|
| $\delta$ = | 7,98 (s; 1 H) |
| | 7,29 (s; 5 H) |
| | 5,18 (s; 2 H) |
| | 4,66 - 4,32 (t; 2 H) |
| | 4,15 - 3,85 ppm (t; 2 H) |
| UV ($CH_2Cl_2$):$\lambda_{max}$ (log $\epsilon$) = | 271 nm (3,85). |

| $C_{13}H_{12}N_4O_4$ (288,30) | | | |
|---|---|---|---|
| Berechnet: | C 54,16 | H 4,20 | N 19,43 |
| Gefunden: | C 53,96 | H 4,17 | N 19,55 |

Stufe 3: Synthese von 1-Benzyl-3-($\beta$-hydroxyethyl)amino-4-nitropyrazol

100 mg (0,35 mmol) N-(1-Benzyl-4-nitro-pyrazolyl)oxazolidin-2-on werden in 10 ml 5 normaler Natronlauge 4 Stunden lang bei 70 Grad Celsius erhitzt. Das Lösungsmittel wird am Rotationsverdampfer im Vakuum abdestilliert und der Rückstand durch Säulenchromatographie an Kieselgel mit Chloroform/Methanol (10 : 1) aufgetrennt.

1. Fraktion:

14 mg (13,9 Prozent der Theorie) N-(1-Benzyl-4-nitropyrazolyl)oxazolidin-2-on mit einem Schmelzpunkt von 120 Grad Celsius (Essigester).

2. Fraktion:

72 mg (78,4 Prozent der Theorie) 1-Benzyl-3-($\beta$-hydroxyethyl)amino-4-nitropyrazol als leuchtend gelbe Kristalle mit einem Schmelzpunkt von 94 Grad Celsius.
60-MHz-$^1$H-NMR ($CDCl_3$):

| | |
|---|---|
| $\delta$ = | 7,70 (s; 1 H) |
| | 7,32 (s; 5 H) |
| | 6,10 ppm (s; 1 H; mit $D_2O$ austauschbar) |
| | 5,05 (s; 2 H) |
| | 3,98 - 3,32 (m; 4 H) |
| | 2,89 - 2,60 ppm (t; 1 H; mit $D_2O$ austauschbar). |
| MS (70 eV): m/e (Prozent) = | 262 (13; $M^+$), 231 (63), 218 (13), 91 (100), 65 (22). |
| UV ($CH_2Cl_2$):$\lambda_{max}$ (log $\epsilon$ ) = | 279 (3,89), 370 nm (3,75). |

| $C_{12}H_{14}N_4O_3$ (262,39) | | | |
|---|---|---|---|
| Berechnet: | C 54,95 | H 5,38 | N 21,36 |
| Gefunden: | C 54,87 | H 5,48 | N 21,45 |

Stufe 4: Reduktion von 1-Benzyl-3-(2'-hydroxyethyl)amino-4-nitropyrazol

250 mg (0,76 mmol) 1-Benzyl-3-(2'-hydroxyethyl)amino-4-nitropyrazol werden mit 4 ml einer 4,4 prozentigen Lösung von Ameisensäure in Methanol und katalytischen Mengen Palladium/Kohlenstoff (10 prozentig) versetzt. Es wird 48 Stunden lang unter Stickstoff bei Raumtemperatur gerührt, sodann der Katalysator abfiltriert und das Lösungsmittel am Rotationsverdampfer im Vakuum abdestilliert. Der Rückstand wird im Vakuum getrocknet. Die Ausbeute ist quantitativ.
60 MHz-$^1$H-NMR ($CDCl_3$):

| | |
|---|---|
| $\delta$ = | 8,02 (s; 4 H; mit $D_2O$ austauschbar) |
| | 7,20 (s; 6 H) |
| | 5,00 (s; 2 H) |
| | 3,90 - 3,15 ppm (m; 4 H) |

**Beispiel 4:** Synthese von 4,5-Diamino-1-methylpyrazolium-hydrogensulfat-hydrat

Stufe 1: Synthese von 3- und 5-Amino-1-methyl-4-nitropyrazol

Zu 2,00 g (15,6 mmol) 3(5)-Amino-4-nitropyrazol in 50 ml 2 normaler Natronlauge werden unter Rühren langsam 4,00 g (31,7 mmol) Dimethylsulfat getropft. Es wird 17 Stunden lang bei Raumtemperatur gerührt. Der entstandene Niederschlag wird abgesaugt und mit Methanol gewaschen.

Ausbeute:

1,54 g (69,5 Prozent der Theorie) 3- und 5-Amino-1-methyl-4-nitropyrazol als Substanzgemisch.

Die chromatographische Auftrennung des Substanzgemisches an einer Kieselgelsäule (l = 100 cm; d = 3 cm) mit Chloroform/Methanol 10 : 1 als Laufmittel ergibt:

1. Fraktion:

680 mg (45,3 Prozent der Theorie) 3-Amino-1-methyl-4-nitropyrazol (Rf-Wert 0,53 $CHCl_3/CH_3OH$ 10 : 1) mit einem Schmelzpunkt von 194 Grad Celsius.

2. Fraktion:

380 mg (24,7 Prozent der Theorie) 5-Amino-1-methyl-4-nitropyrazol (Rf-Wert 0,37 $CHCl_3/CH_3OH$ 10 : 1) mit einem Schmelzpunkt von 266 Grad Celsius.

Stufe 2: Synthese von 4,5-Diamino-1-methylpyrazoliumhydrogensulfat-hydrat

200 mg (1,41 mmol) 4-Amino-1-methyl-5-nitropyrazol werden in 50 ml absolutem Methanol mit katalytischen Mengen Palladium/Kohlenstoff bei Raumtemperatur und 30 bar hydriert. Nach 17 Stunden ist die Hydrierung beendet. Aus der filtrierten Lösung fällt bei Zugabe von 135 mg (1,41 mmol) konzentrierter Schwefelsäure ein farbloser Niederschlag aus, der abgesaugt und aus Wasser umkristallisiert wird.

Ausbeute:

150 mg (46,6 Prozent der Theorie) 4,5-Diamino-1-methylpyrazolium-hydrogensulfat-hydrat als farblose Kristalle mit einem Schmelzpunkt von 200 - 201 Grad Celsius (Zers.) (Wasser).
60-MHz-$^1$H-NMR( $D_6$ -DMSO):
δ = 7,25 (s; 1 H)
7,18 - 6,20 (s; 8 H; mit $D_2O$ austauschbar)
3,61 ppm (s; 3 H)

| $C_4H_{12}N_4O_5S$ (228,23) | | | |
|---|---|---|---|
| Berechnet:<br>Gefunden: | C 21,05<br>C 20,86 | H 5,30<br>H 5,35 | N 24,55<br>N 24,29 |

**Beispiel 5:** Synthese von 3,4-Diamino-1-methylpyrazol

90 mg (0,63 mmol) 3-Amino-1-methyl-4-nitropyrazol werden in 80 ml absolutem Methanol mit katalytischen Mengen Palladium/Kohlenstoff bei Raumtemperatur und 50 bar hydriert. Nachdem das Lösungsmittel am Rotationsverdampfer im Vakuum auf 1/3 des ursprünglichen Volumens eingeengt wurde, fällt aus der filtrierten Lösung bei Zugabe von 124 mg (1,26 mmol) konzentrierter Schwefelsäure ein weißer Niederschlag aus, der abgesaugt und getrocknet wird. Nach 17 Stunden ist die Hydrierung beendet.

Ausbeute:

100 mg (75,5 Prozent der Theorie) 3,4-Diamino-1-methylpyrazolium-hydrogensulfat als weiße Kristalle mit einem Schmelzpunkt von 214 - 215 Grad Celsius (Methanol).
300-MHz-$^1$H-NMR ( $D_6$ -DMSO):
δ = 7,55 (s; 1 H)
7,67 - 7,20 (m; 6 H; mit $D_2O$ austauschbar)
3,60 ppm (s; 3 H)

| $C_4H_8N_4$ * 1,1 $H_2SO_4$ (220,01) | | | |
|---|---|---|---|
| Berechnet:<br>Gefunden: | C 21,84<br>C 21,83 | H 4,67<br>H 4,63 | N 25,46<br>N 25,18 |

**Beispiel 6:** Synthese von 4-Amino-1-methyl-3-methylamino-pyrazoliumhydrogensulfat

Stufe 1: Synthese von 3-Trifluoracetylamino-1-methyl-4-nitropyrazol

Methode A

7,50 ml Trifluoracetanhydrid werden portionsweise mit 1,50 g (10,6 mmol) 3-Amino-1-methyl-4-nitropyrazol versetzt. Nach 17 stündigem Rühren bei Raumtemperatur wird das Lösungsmittel am Rotationsverdampfer im Vakuum abdestilliert und der Rückstand mit Hexan/Ether versetzt, wobei ein weißer Niederschlag auskristallisiert.

Ausbeute:

2,40 g (95,4 Prozent der Theorie) 3-Trifluoracetylamino-1-methyl-4-nitropyrazol als weiße Nadeln mit einem Schmelzpunkt von 104 Grad Celsius (Ether).
60-MHz-$^1$H-NMR ($CDCl_3$):
δ = 9,72 (s; 1 H; mit $D_2O$ austauschbar)
8,12 (s; 1 H)
3,98 ppm (s; 3 H).

MS (70 eV): m/e (Prozent) = 238 (81; $M^+$), 169 (100), 152 (63), 125 (13), 69 (31), 52 (37), 42 (66).
UV ($CH_2Cl_2$):$\lambda_{max}$ (log $\epsilon$ ) = 292 nm (3,89).

| $C_6H_5F_3N_4O_3$ (238,12) | | | |
|---|---|---|---|
| Berechnet: | C 30,26 | H 2,11 | N 23,53 |
| Gefunden: | C 30,21 | H 1,94 | N 23,51 |

Methode B

5,00 ml konzentrierte Schwefelsäure werden portionsweise mit 1,00 g (5,21 mmol) 3-Trifluoracetylamino-1-methylpyrazol versetzt. Danach wird 1 ml 100 prozentige Nitriersäure zugetropft und 17 Stunden lang bei Raumtemperatur gerührt. Die Lösung wird auf 40 g Eis gegossen, wobei ein farbloser Niederschlag auskristallisiert, der abgesaugt und getrocknet wird.

Ausbeute:

270 mg (22,2 Prozent der Theorie) 3-Trifluoracetylamino-1-methyl-4-nitropyrazol mit einem Schmelzpunkt von 104 Grad Celsius (Ether).
Die Mutterlauge wird mit konzentriertem Ammoniak neutralisiert und 24 Stunden lang mit Ether in einem Rotationsperforator extrahiert. Beim Einengen der organischen Phase können weitere 220 mg (18,1 Prozent der Theorie) 3-Trifluoracetylamino-1-methyl-4-nitropyrazol isoliert werden.

Stufe 2: Synthese von 1-Methyl-3-methylamino-4-nitropyrazol

1,00 g (4,20 mmol) 3-Trifluoracetylamino-1-methyl-4-nitropyrazol werden mit 2,12 g (16,8 mmol) Methyliodid in 10 ml absolutem Aceton auf 50 Grad Celsius erhitzt. Sodann werden 940 mg (16,8 mmol) gepulvertes Kaliumhydroxid zugegeben und das Reaktionsgemisch 5 Minuten lang zum Sieden erhitzt. Das Lösungsmittel wird am Rotationsverdampfer im Vakuum abdestilliert und der Rückstand durch Säulenchromatographie an Kieselgel mit Ether/Toluol (5 : 1) aufgetrennt.

1. Fraktion:

370 mg (62,0 Prozent der Theorie) 1-Methyl-3-methylamino-4-nitropyrazol als leuchtend gelbe Kristalle mit einem Schmelzpunkt von 176 Grad Celsius (Ether).
60-MHz-$^1$H-NMR ( $D_6$ -DMSO):
δ = 8,38 (s; 1 H)
6,40 (s; 1 H; mit $D_2O$ austauschbar)
3,68 (s; 3 H; Methylgruppe am Pyrazolring)
2,82 - 2,72 ppm (d; 3 H; J = 6 Hz; - NH - $CH_3$).
MS (70 eV): m/e (Prozent) = 156 (53; $M^+$), 138 (15), 109 (24), 71 (53), 68 (56), 52 (44), 42 (100).
UV ($CH_2Cl_2$):$\lambda_{max}$ (log $\epsilon$ ) = 280 (3,85), 373 nm (3,75).

| $C_5H_8N_4O_2$ (156,14) | | | |
|---|---|---|---|
| Berechnet: | C 38,46 | H 5,16 | N 35,88 |
| Gefunden: | C 38,21 | H 5,22 | N 35,75 |

Als 2. Fraktion konnten 210 mg (35,2 Prozent der Theorie) 3-Amino-1-methyl-4-nitropyrazol isoliert werden.

Stufe 3: Synthese von 4-Amino-1-methyl-3-methylaminopyrazolium-hydrogensulfat

500 mg (3,20 mmol) 1-Methyl-3-methylamino-4-nitropyrazol werden in 50 ml absolutem Methanol mit katalytischen Mengen Palladium/Kohlenstoff bei Raumtemperatur und 30 bar hydriert. Nach 17 Stunden ist die Hydrierung beendet, aus der filtrierten Lösung fällt bei Zugabe von 314 mg (3,20 mmol) konzentrierter

Schwefelsäure ein blaßorangener Niederschlag aus, der abgesaugt und getrocknet wird.

Ausbeute:

590 mg (82,2 Prozent der Theorie) 4-Amino-1-methyl-3-methylaminopyrazolium-hydrogensulfat als blaßorangene Kristalle mit einem Schmelzpunkt von 209 Grad Celsius.
60-MHz-$^1$H-NMR ( $D_6$ -DMSO):
δ = 8,07 (s; 5 H; mit $D_2O$ austauschbar)
7,52 (s; 1 H)
3,58 (s; 3 H; Methylgruppe am Pyrazolring)
2,70 ppm (s; 3 H)

| $C_5H_{12}N_4O_4S$ (224,24) | | | |
|---|---|---|---|
| Berechnet:<br>Gefunden: | C 26,78<br>C 26,42 | H 5,39<br>H 5,38 | N 24,99<br>N 24,91 |

**Beispiel 7:** Synthese von 4-Amino-5-(N,N-dimethylamino)-1-methylpyrazolium-dihydrogensulfat

Stufe 1: Umsetzung eines Gemisches aus 3- und 5-Trifluoracetylamino-1-methyl-4-nitropyrazol mit Methyliodid

3,94 g (16,5 mmol) eines Gemisches aus 3- und 5-Trifluoracetylamino-1-methyl-4-nitropyrazol werden mit 8,48 g (16,8 mmol) Methyljodid in 40 ml absolutem Aceton auf 50 Grad Celsius erhitzt, sodann werden 3,77 g (16,8 mmol) gepulvertes Kaliumhydroxid zugegeben und die Lösung 5 Minuten lang zum Sieden erhitzt. Das Lösungsmittel wird am Rotationsverdampfer im Vakuum abdestilliert und der Rückstand durch Säulenchromatographie an Kieselgel mit Ether/Toluol (5 : 1) aufgetrennt.

1. Fraktion:

980 mg (34,8 Prozent der Theorie) 5-(N,N-Dimethylamino)-1-methyl-4-nitropyrazol als gelbes Öl, das im Kugelrohr bei 50 Grad Celsius/0,04 Torr destilliert wird.
60-MHz-$^1$H-NMR ($CDCl_3$):
δ = 7,95 (s; 1 H)
3,72 (s; 3 H)
2,89 ppm (s; 6 H)
MS (70 eV): m/e (Prozent) = 170 (22; $M^+$), 153 (31), 146 (21), 125 (90), 123 (62), 108 (55), 82 (70), 70 (99), 66 (92), 42 (100).

| $C_6H_{10}N_4O_2$ (170,17) | | | |
|---|---|---|---|
| Berechnet:<br>Gefunden: | C 42,35<br>C 42,14 | H 5,92<br>H 5,99 | N 32,92<br>N 32,75 |

2. Fraktion:

1,38 mg (53,4 Prozent der Theorie) 1-Methyl-3-methylamino-4-nitropyrazol als leuchtend gelbe Kristalle mit einem Schmelzpunkt von 176 Grad Celsius (Ether).

Stufe 2: Synthese von 4-Amino-5-(N,N-dimethylamino)-1-methylpyrazolium-dihydrogensulfat

560 mg (3,29 mmol) 5-(N,N-Dimethylamino)-1-methyl-4-nitropyrazol werden in 75 ml absolutem Methanol mit katalytischen Mengen Palladium/Kohlenstoff bei Raumtemperatur und 30 bar hydriert. Nach 17 Stunden ist die Hydrierung beendet. Es werden 645 mg (6,58 mmol) konzentrierte Schwefelsäure zugesetzt und der Katalysator abfiltriert. Das Lösungsmittel wird abdestilliert und der Rückstand mit 2-Propanol

versetzt, wobei ein farbloser Niederschlag auskristallisiert.

Ausbeute:

300 mg (27,1 Prozent der Theorie) 4-Amino-5-(N,N-dimethylamino)-1-methylpyrazolium-dihydrogensulfat mit einem Schmelzpunkt von 139 Grad Celsius (2-Propanol).
60-MHz-$^1$H-NMR ( $D_6$ -DMSO):
δ = 9,78 (s; 6 H; mit $D_2O$ austauschbar)
7,35 (s; 1 H)
3,61 (s; 3 H)
2,78 ppm (s; 6 H)

| $C_6H_{16}N_4O_8S_2$ (336,35) | | | |
|---|---|---|---|
| Berechnet: | C 21,42 | H 4,79 | N 16,66 |
| Gefunden: | C 21,11 | H 4,72 | N 16,37 |

**Beispiele für Haarfärbemittel**

**Beispiel 8:** Haarfärbemittel in Gelform

| | |
|---|---|
| 0,50 g | 3,4-Diaminopyrazol-dihydrochlorid |
| 0,50 g | 5-Amino-2-methylphenol |
| 0,15 g | Natriumsulfit, wasserfrei |
| 5,00 g | Laurylalkohol-diglykolethersulfat-Natriumsalz (28 prozentige wäßrige Lösung) |
| 1,00 g | Hydroxyethylcellulose, hochviskos |
| 10,00 g | Ammoniak (22 prozentige wäßrige Lösung) |
| 82,85 g | Wasser |
| 100,00 g | |

50 g des vorstehenden Haarfärbemittels werden kurz vor dem Gebrauch mit 50 g Wasserstoffperoxidlösung (6 prozentig) vermischt und das Gemisch anschließend auf blonde Naturhaare aufgetragen. Nach einer Einwirkungszeit von 30 Minuten bei 40 Grad Celsius wird das Haar mit Wasser gespült und getrocknet. Das Haar hat eine intensive leuchtend rotorange Färbung erhalten.

**Beispiel 9:** Haarfärbemittel in Gelform

| | |
|---|---|
| 0,35 g | 4,5-Diamino-1-methylpyrazol-dihydrochlorid |
| 0,27 g | 3-Aminophenol |
| 0,30 g | Ascorbinsäure |
| 15,00 g | Ölsäure |
| 7,00 g | Isopropanol |
| 10,00 g | Ammoniak (22 prozentige wäßrige Lösung) |
| 67,08 g | Wasser |
| 100,00 g | |

Man vermischt kurz vor dem Gebrauch 50 g dieses Haarfärbemittels mit 50 g Wasserstoffperoxidlösung (6 prozentig) und läßt das Gemisch 30 Minuten lang bei 40 Grad Celsius auf weiße menschliche Haare einwirken. Sodann wird das Haar mit Wasser gespült und getrocknet. Das Haar ist in einem leuchtenden roten Farbton gefärbt.

**Beispiel 10:** Haarfärbemittel in Cremeform

| | |
|---|---|
| 1,00 g | 4-Amino-5-(N,N-dimethylamino)-1-methylpyrazoliumdihydrogensulfat nach Beispiel 7 |
| 1,10 g | 1-Naphthol |
| 15,00 g | Cetylalkohol |
| 0,30 g | Natriumsulfit, wasserfrei |
| 3,50 g | Laurylalkohol-diglykolethersulfat-Natriumsalz (28 prozentige wäßrige Lösung) |
| 3,00 g | Ammoniak (22 prozentige wäßrige Lösung) |
| 76,10 g | Wasser |
| 100,00 g | |

50 g dieses Haarfärbemittels werden kurz vor dem Gebrauch mit 50 g Wasserstoffperoxidlösung (6 prozentig) vermischt. Anschließend trägt man das Gemisch auf blonde Naturhaare auf und läßt es 30 Minuten lang bei 40 Grad Celsius einwirken. Danach wird das Haar mit Wasser gespült und getrocknet. Das Haar hat eine intensive lachsrote Färbung erhalten.

**Beispiel 11:** Haarfärbelösung

| | |
|---|---|
| 0,50 g | 3,4-Diaminopyrazol-dihydrochlorid |
| 0,50 g | 2-Amino-5-methylphenol |
| 0,50 g | 2-Amino-4-(2'-hydroxyethyl)amino-anisolsulfat |
| 0,05 g | 1-Naphthol |
| 10,00 g | Laurylalkohol-diglykolethersulfat-Natriumsalz (28 prozentige wäßrige Lösung) |
| 10,00 g | Ammoniak (22 prozentige wäßrige Lösung) |
| 78,45 g | Wasser |
| 100,00 g | |

Man vermischt kurz vor dem Gebrauch 50 g des vorstehenden Haarfärbemittels mit 50 g Wasserstoffperoxidlösung (6 prozentig) und läßt die Mischung 30 Minuten lang bei 40 Grad Celsius auf blonde Naturhaare einwirken. Sodann wird das Haar mit Wasser gespült und getrocknet. Das Haar ist in einem modischen dunkelbraunen Palisanderton gefärbt.

**Beispiel 12:** Färbemittel in Gelform

| | |
|---|---|
| 1,00 g | 4,5-Diamino-1-methylpyrazol-dihydrochlorid |
| 2,00 g | 2,5-Diaminotoluolsulfat |
| 1,50 g | 2-Amino-4-(2'-hydroxyethyl)amino-anisolsulfat |
| 0,10 g | 1-(2'-Ureidoethyl)amino-4-nitrobenzol |
| 0,15 g | Natriumsulfit, wasserfrei |
| 2,50 g | Laurylalkohol-diglykolethersulfat-Natriumsalz (28 prozentige wäßrige Lösung) |
| 0,80 g | Hydroxyethylcellulose, hochviskos |
| 6,00 g | Ammoniak (22 prozentige wäßrige Lösung) |
| 85,95 g | Wasser |
| 100,00 g | |

50 g des obigen Haarfärbemittels werden kurz vor dem Gebrauch mit 50 g Wasserstoffperoxidlösung (6 prozentig) vermischt und die Mischung anschließend auf blonde Naturhaare aufgebracht. Nach einer Einwirkungszeit von 30 Minuten bei 40 Grad Celsius wird mit Wasser gespült und getrocknet. Das Haar hat eine schwarze Färbung erhalten.

**Beispiele 13 bis 27:** Haarfärbelösungen

Man verwendet die Lösung nach Beispiel 8 und ersetzt das 3,4-Diaminopyrazoldihydrochlorid mengengleich durch andere Pyrazolderivate ("Entwickler") der Formel (I) aus den Beispielen 1 - 7 sowie das 5-Amino-2-methyl-phenol mengengleich durch die in der Tabelle 1 angegebenen "Kuppler":

Tabelle 1:

| Beispiel | Entwickler der Formel (I) aus Beispiel | Kuppler | Farbe |
|---|---|---|---|
| 13 | 1 | 5-Amino-2-methylphenol | leuchtend rotorange |
| 14 | 2 | 5-Amino-2-methylphenol | rot |
| 15 | 3 | 5-Amino-2-methylphenol | orange |
| 16 | 6 | 5-Amino-2-methylphenol | orange |
| 17 | 7 | 5-Amino-2-methylphenol | ziegelrot |
| 18 | 1 | 3-Aminophenol | leuchtend rot |
| 19 | 3 | 3-Aminophenol | rot |
| 20 | 6 | 3-Aminophenol | rot |
| 21 | 7 | 3-Aminophenol | rot |

| Beispiel | Entwickler der Formel (I) aus Beispiel | Kuppler | Farbe |
|---|---|---|---|
| 22 | 1 | 2-Amino-4-(2'-hydroxyethyl)aminoanisolsulfat | violett |
| 23 | 2 | 2-Amino-4-(2'-hydroxyethyl)aminoanisolsulfat | graublau |
| 24 | 3 | 2-Amino-4-(2'-hydroxyethyl)aminoanisolsulfat | grauviolett |
| 25 | 5 | 2-Amino-4-(2'-hydroxyethyl)aminoanisolsulfat | grauviolett |
| 26 | 6 | 2-Amino-4-(2'-hydroxyethyl)aminoanisolsulfat | violett |
| 27 | 7 | 2-Amino-4-(2'-hydroxyethyl)aminoanisolsulfat | blauviolett |

Alle in der vorliegenden Patentanmeldung angegebenen Prozentzahlen stellen, sofern nicht anders angegeben, Gewichtsprozente dar.

## Patentansprüche

1. Mittel zur oxidativen Färbung von Haaren auf der Basis einer Entwicklersubstanz-Kupplersubstanz-Kombination, dadurch gekennzeichnet, daß es als Entwicklersubstanz ein Diaminopyrazol der allgemeinen Formel (I)

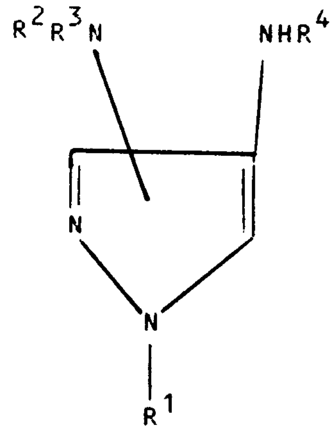

(I),

in der $R^1$, $R^2$ und $R^4$ gleich oder verschieden sind und Wasserstoff, Alkyl mit 1 bis 4 Kohlenstoffatomen, Hydroxyalkyl mit 2 bis 4 Kohlenstoffatomen, Benzyl oder Phenyl bedeuten und $R^3$ Wasserstoff, Alkyl mit 1 bis 4 Kohlenstoffatomen oder Hydroxyalkyl mit 2 bis 4 Kohlenstoffatomen darstellt,
oder dessen physiologisch verträgliche,
wasserlösliche Salze enthält.

2. Mittel nach Anspruch 1, dadurch gekennzeichnet, daß das Diaminopyrazol ausgewählt ist aus 3(5),4-Diaminopyrazol, 4,5-Diamino-1-methylpyrazol oder 4,5-Diamino-1-benzylpyrazol.

3. Mittel nach Anspruch 1 oder 2, dadurch gekennzeichnet, daß die Entwicklersubstanz der Formel (I) in einer Menge von 0,01 bis 3,0 Gewichtsprozent enthalten ist.

4. Mittel nach einem der Ansprüche 1 bis 3, dadurch gekennzeichnet, daß die Kupplersubstanz ausgewählt ist aus 1-Naphthol, Resorcin, 4-Chlorresorcin, 4,6-Dichlorresorcin, 2-Methylresorcin, 2-Amino-4-(2'-hydroxyethyl)amino-anisol, 5-Amino-2-methylphenol, 2,4-Diaminophenoxyethanol, 4-Amino-2-hydroxyphenoxyethanol, 3-Aminophenol, 3-Amino-2-methylphenol, 4-Hydroxy-1,2-methylendioxybenzol, 4-Amino-1,2-methylendioxybenzol, 4-(2'-Hydroxyethyl)amino-1,2-methylendioxybenzol, 2,4-Diaminophenetol, 2,4-Diamino-5-methylphenetol, 2,4-Diaminobenzylalkohol, m-Phenylendiamin, 2,4-Diaminophenylethylalkohol, 4-Hydroxyindol, 3-Amino-5-hydroxy-2,6-dimethoxypyridin und 3,5-Diamino-2,6-dimethoxypyridin.

5. Mittel nach einem der Ansprüche 1 bis 4, dadurch gekennzeichnet, daß die Gesamtmenge der EntwicklerKupplersubstanz-Kombinationen 0,1 bis 5,0 Gewichtsprozent, vorzugsweise 0,5 bis 4,0 Gewichtsprozent, beträgt.

6. Mittel nach einem der Ansprüche 1 bis 5, dadurch gekennzeichnet, daß es eine Farbkomponente enthält, die ausgewählt ist aus 6-Amino-2-methylphenol, 2-Amino-5-methylphenol, Diamond Fuchsine (C.I. 42 510), Leather Ruby HF (C.I. 42 520), 2-Nitro-1,4-diaminobenzol, 2-Amino-4-nitrophenol, 2-Amino-5-nitrophenol, 2-Amino-4,6-dinitrophenol, 2-Amino-5-(2'-hydroxyethyl)amino-nitrobenzol, 2-Methylamino-5-bis-(2'-hydroxyethyl)aminonitrobenzol, Acid Brown 4 (C.I. 14 805), 1,4-Diaminoanthrachinon und 1,4,5,8-Tetraaminoanthrachinon.

7. 3,4-Diamino-1-methylpyrazol

**8.** Diaminopyrazolderivat der allgemeinen Formel (II)

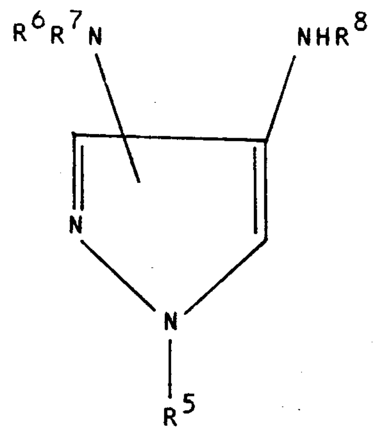

(II),

in der $R^5$ ein Benzylrest ist, $R^6$ und $R^8$ gleich oder verschieden sind und Wasserstoff, Alkyl mit 1 bis 4 Kohlenstoffatomen, Hydroxyalkyl mit 2 bis 4 Kohlenstoffatomen, Benzyl oder Phenyl bedeuten und $R^7$ Wasserstoff, Alkyl mit 1 bis 4 Kohlenstoffatomen oder Hydroxyalkyl mit 2 bis 4 Kohlenstoffatomen darstellt.

**9.** Diaminopyrazolderivat gemäß Anspruch 8, dadurch gekennzeichnet, daß es das 4,5-Diamino-1-benzylpyrazol ist.

**10.** Diaminopyrazolderivat gemäß Anspruch 8, dadurch gekennzeichnet, daß es das 3,4-Diamino-1-benzylpyrazol ist.

**11.** Diaminopyrazolderivat gemäß Anspruch 8, dadurch gekennzeichnet, daß es das 4-Amino-1-benzyl-3-(2'-hydroxyethyl)amino-pyrazol ist.

**12.** Diaminopyrazolderivat gemäß Anspruch 8, dadurch gekennzeichnet, daß es das 4-Amino-1-benzyl-3-benzylaminopyrazol ist.

**13.** Diaminopyrazolderivat der allgemeinen Formel (III)

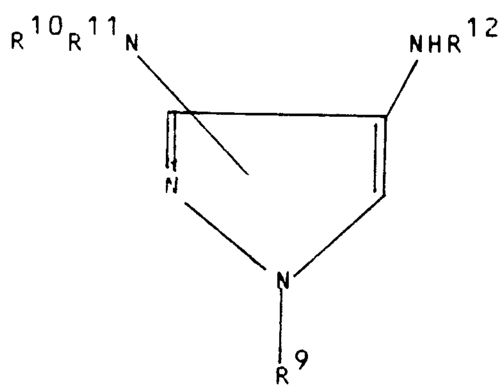

(III),

in der $R^9$ ein Methylrest ist, $R^{10}$ und $R^{12}$ gleich oder verschieden sind und Wasserstoff, Alkyl mit 1 bis 4 Kohlenstoffatomen, Hydroxyalkyl mit 2 bis 4 Kohlenstoffatomen, Benzyl oder Phenyl bedeuten und $R^{11}$ Wasserstoff, Alkyl mit 1 bis 4 Kohlenstoffatomen, Hydroxyalkyl mit 2 bis 4 Kohlenstoffatomen darstellt, unter der Voraussetzung, daß mindestens einer der Reste $R^{10}$ bis $R^{12}$ von Wasserstoff verschieden ist.

**14.** Diaminopyrazolderivat gemäß Anspruch 13, dadurch gekennzeichnet, daß es das 4-Amino-1-methyl-3-methylaminopyrazol ist.

**15.** Diaminopyrazolderivat gemäß Anspruch 13, dadurch gekennzeichnet, daß es das 4-Amino-1-methyl-5-N,N-dimethylaminopyrazol ist.

**Claims**

**1.** Agent for the oxidative colouring of hair based on a combination of a developer substance and coupler substance, characterized in that it contains a diaminopyrazole of the general formula (I)

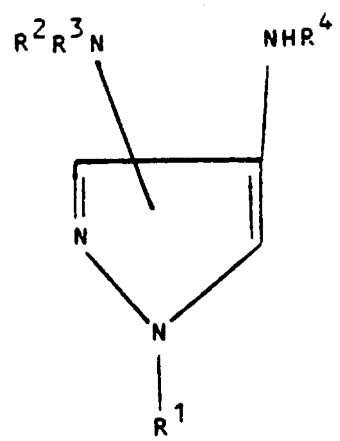

(I),

wherein $R^1$, $R^2$ and $R^4$ are either similar or different and signify hydrogen, alkyl having 1 to 4 carbon atoms, hydroxyalkyl having 2 to 4 carbon atoms, benzyl or phenyl and $R^3$ is hydrogen, alkyl having 1 to 4 carbon atoms or hydroxyalkyl having 2 to 4 carbon atoms, or physiologically-compatible water-soluble salts thereof, as the developer substance.

**2.** Agent according to claim 1, characterized in that the diaminopyrazole is selected from 3(5),4-diaminopyrazole, 4,5-diamino-1-methylpyrazole or 4,5-diamino-1-benzylpyrazole.

**3.** Agent according to claim 1 or 2, characterized in that the developer substance according to formula (I) is present in an amount of 0.01 to 3.0 percent by weight.

**4.** Agent according to any one of claims 1 to 3, characterized in that the coupler substance is selected from 1-naphtol, resorcinol, 4-chloro-resorcinol, 4,6-dichloro-resorcinol, 2-methyl-resorcinol, 2-amino-4-(2'-hydroxyethyl)amino-anisole, 5-amino-2-methyl-phenol, 2,4-diaminophenoxyethanol, 4-amino-2-hydroxyphenoxyethanol, 3-aminophenol, 3-amino-2-methylphenol, 4-hydroxy-1,2-methylenedioxybenzene, 4-amino1,2-methylenedioxybenzene, 4-(2'-hydroxyethyl)amino-1,2-methylenedioxybenzene, 2,4-diaminophenetol, 2,4-diamino-5-methylphenetol, 2,4-diaminobenzylalcohol, m-phenylenediamine, 2,4-diaminophenylethylalcohol, 4-hydroxyindole, 3-amino-5-hydroxy-2,6-dimethoxypyridine and 3,5-diamino-2,6-dimethoxypyridine.

**5.** Agent according to any one of claims 1 to 4, characterized in that the total amount of developer-coupler-substance-combination is 0.1 to 5.0 percent by weight, preferably 0.5 to 4.0 percent by weight.

**6.** Agent according to any one of claims 1 to 5, characterized in that it contains a colouring component selected from the group consisting of 6-amino-2-methylphenol, 2-amino-5-methylphenol, diamond fuchsine (C.I. 42 510), leather ruby HF (C.I. 42 520), 2-nitro-1,4-diaminobenzene, 2-amino-4-nitrophenol, 2-amino-5-nitrophenol, 2-amino-4,6-dinitrophenol, 2-amino-5-(2'-hydroxyethyl)amino-nitrobenzene, 2-methylamino-5-bis-(2'-hydroxyethyl)aminonitrobenzene, acid brown 4 (C.I. 14 805), 1,4-diaminoanthraquinone and 1,4,5,8-tetraaminoanthraquinone.

**7.** 3,4-diamino-1-methylpyrazole.

**8.** Diaminopyrazole derivative of the general formula (II)

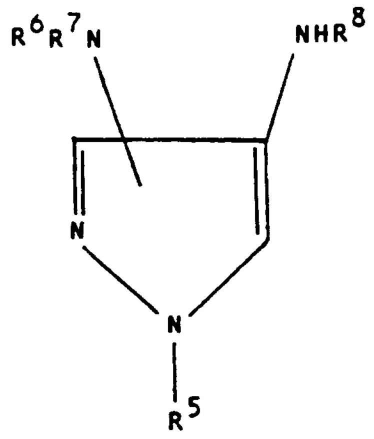

(II),

wherein $R^5$ is a benzyl residue, $R^6$ and $R^8$ are the same or different and signify hydrogen, alkyl having 1 to 4 carbon atoms, hydroxyalkyl having 2 to 4 carbon atoms, benzyl or phenyl, and $R^7$ is hydrogen, alkyl having 1 to 4 carbon atoms or hydroxyalkyl having 2 to 4 carbon atoms.

**9.** Diaminopyrazole derivative according to claim 8, characterized in that it is the 4,5-diamino-1-benzyl-pyrazole.

**10.** Diaminopyrazole derivative according to claim 8, characterized in that it is the 3,4-diamino-1-benzyl-pyrazole.

**11.** Diaminopyrazole derivative according to claim 8, characterized in that it is the 4-amino-1-benzyl-3-(2'-hydroxyethyl)amino-pyrazole.

**12.** Diaminopyrazole derivative according to claim 8, characterized in that it is the 4-amino-1-benzyl-3-benzylaminopyrazole.

**13.** Diaminopyrazole derivative of the general formula (III)

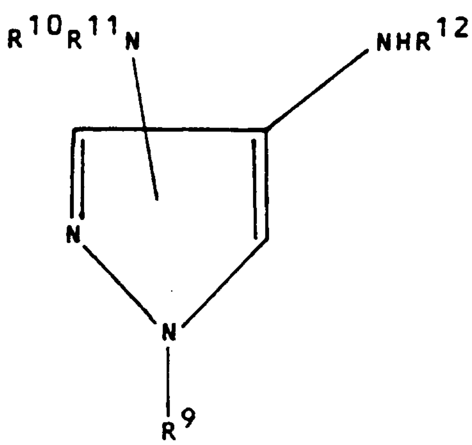

(III),

wherein $R^9$ is a methyl residue, $R^{10}$ and $R^{12}$ are similar or different and signify hydrogen, alkyl having 1 to 4 carbon atoms, hydroxyalkyl having 2 to 4 carbon atoms, benzyl or phenyl, and $R^{11}$ is hydrogen, alkyl with 1 to 4 carbon atoms, hydroxyalkyl having 2 to 4 carbon atoms, on the condition that at least one of the $R^{10}$ to $R^{12}$ residue is not hydrogen.

**14.** Diaminopyrazole derivative according to claim 13, characterized in that it is the 4-amino-1-methyl-3-methylaminopyrazole.

**15.** Diaminopyrazole derivative according to claim 13, characterized in that it is the 4-amino-1-methyl-5-N,N-dimethylaminopyrazole.

**Revendications**

**1.** Agent de coloration oxydant pour les cheveux à base d'une combinaison d'un copulant et d'un révélateur, caractérisé en ce qu'il contient comme révélateur un diamino-pyrazole de formule générale (I)

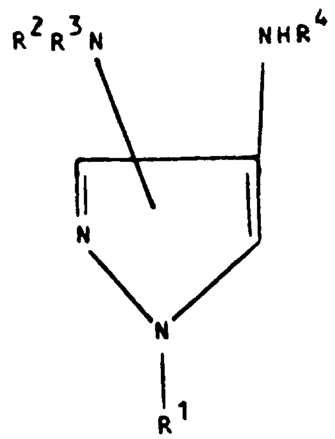

(I),

dans laquelle $R^1$, $R^2$ et $R^4$ sont identiques ou différents et représentent l'hydrogène, un radical alkyle avec l à 4 atomes de carbone, hydroxyalkyle avec 2 à 4 atomes de carbone, benzyle ou phényle, et $R^3$ représente l'hydrogène, un radical alkyle avec 1 à 4 atomes de carbone ou hydroxyalkyle avec 2 à 4 atomes de carbone, ou contient ses sels solubles dans l'eau tolérés physiologiquement.

**2.** Agent selon la revendication 1, carctérisé en ce que le diaminopyrazole est choisi parmi le 3(5),4-diaminopyrazole, le 4,5-diamino-1-méthyl-pyrazole ou le 4,5-diamino-1-benzyl-pyrazole.

**3.** Agent selon la revendication 1 ou 2, caractérisé en ce que le révélateur de formule (I) est présent à raison de 0,01 à 3,0 % en masse.

**4.** Agent selon l'une des revendications 1 à 3, caractérisé en ce que le copulant est choisi parmi le 1-naphtol, la résorcine, la 4-chloro-résorcine, la 4,6-dichloro-résorcine, la 2-méthyl-résorcine, le 2-amino-4-(2'-hydroxyéthyl)amino-anisol,le 5-amino-2-méthyl-phénol, le 2,4-diamino-phénoxy-éthanol, le 4-amino-2-hydroxy-phénoxy-éthanol, le 3-amino-phénol, le 3-amino-2-méthyl-phénol, le 4-hydroxy-1,2-méthylène-dioxy-benzène, le 4-amino-1,2-méthylène-dioxy-benzène, le 4-(2'-hydroxyéthyl)amino-1,2-méthylène-dioxy-benzène, le 2,4-diamino-phénétol, le 2,4-diamino-5-méthyl-phénétol, le 2,4-diamino-benzyl-alcool, la m-phénylène-diamine, le 2,4-diamino-phényl-éthyl-alcool, le 4-hydroxy-indole, la 3-amino-5-hydroxy-2,6-diméthoxy-pyridine, et la 3,5-diamino-2,6-diméthoxy-pyridine.

**5.** Agent selon l'une des revendications 1 à 4, caractérisé en ce que la quantité totale de combinaisons copulant-révélateur atteint 0,1 à 5,0 % en masse, de préférence 0,5 à 4,0 % en masse.

**6.** Agent selon l'une des revendications 1 à 5, caractérisé en ce qu'il contient un composant colorant qui est choisi parmi le 6-amino-2-méthyl-phénol, le 2-amino-5-méthyl-phénol, la fuchsine diamant (C.I. 42 510), le "Leather Ruby HF" (C.I. 42 520), le 2-nitro-1,4-diamino-benzène,le 2-amino-4-nitro-phénol, le 2-amino-5-nitro-phénol, le 2-amino-4,6-dinitrophénol, le 2-amino-5-(2'-hydroxy-éthyl)amino-nitro-benzène, le 2-méthylamino-5-bis-(2'-hydroxyéthyl)-amino-nitro-benzéne, le brun acide 4 (C.I. 14 805), la 1,4-diamino-anthraquinone, et le 1,4,5,8-tétra-amino-anthraquinone.

**7.** Le 3,4-diamino-1-méthyl-pyrazole.

**8.** Dérivé de diamino-pyrazole de formule générale (II)

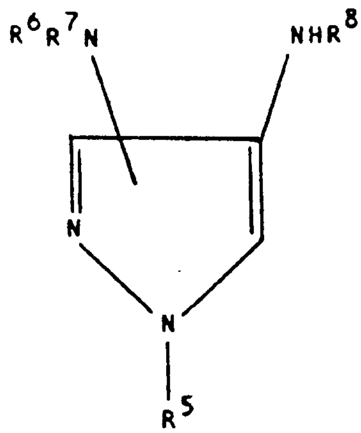

(II),

dans laquelle $R^5$ est un reste benzyle, $R^6$ et $R^8$ sont identiques ou différents, et représentent l'hydrogène, un groupe alkyle avec 1 à 4 atomes de carbone, hydroxy-alkyle avec 2 à 4 atomes de carbone, benzyle ou phényle, et $R^7$ représente l'hydrogène, un groupe alkyle avec 1 à 4 atomes de carbone, hydroxy-alkyle avec 2 à 4 atomes de carbone.

**9.** Dérivé de diamino-pyrazole selon la revendication 8, caractérisé en ce qu'il est le 4,5-diamino-1-benzyle-pyrazole.

**10.** Dérivé de diamino-pyrazole selon la revendication 8, caractérisé en ce qu'il est le 3,4-diamino-1-benzyle-pyrazole.

**11.** Dérivé de diamino-pyrazole selon la revendication 8, caractérisé en ce qu'il est le 4-amino-1-benzyle-3-(2'-hydroxyéthyl)amino-pyrazole.

**12.** Dérivé de diamino-pyrazole selon la revendication 8, caractérisé en ce qu'il est le 4-amino-1-benzyle-3-benzylamino-pyrazole.

**13.** Dérivé de diamino-pyrazole de formule générale (III)

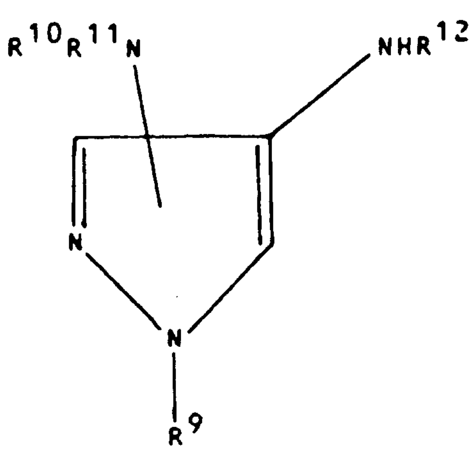

(III),

dans laquelle $R^9$ est un reste méthyle, $R^{10}$ et $R^{12}$ sont identiques ou différents et représentent l'hydrogène, un groupe alkyle avec 1 à 4 atomes de carbone, hydroxy-alkyle avec 2 à 4 atomes de carbone, benzyle ou phényle et $R^{11}$ est l'hydrogène, un groupe alkyle avec 1 à 4 atomes de carbone,

hydroxy-alkyle avec 2 à 4 atomes de carbone,sous la condition qu'au moins un des restes $R^{10}$ à $R^{12}$ ne soit pas l'hydrogène.

**14.** Dérivé de diamino-pyrazole selon la revendication 13, caractérisé en ce qu'il est le 4-Amino-1-méthyl-3-méthylamino-pyrazole.

**15.** Dérivé de diamino-pyrazole selon la revendication 13, caractérisé en ce qu'il est le 4-Amino-1-méthyl-5-N,N-diméthylamino-pyrazole.